# DEMANDE DE BREVET EUROPEEN

(11) **EP 0 646 579 A1**
(43) Date de publication de la demande: **05.04.1995**
(21) Numéro de dépôt: 94402170.8
(22) Date de dépôt: 29.09.1994
(51) Int. Cl.: C07D 277/82, C07D 263/58, C07D 235/20, C07D 221/04, C07D 241/38, C07D 471/04, A61K 31/425, A61K 31/42, A61K 31/495

(54) **Dérivés d'indane cétoniques et de leurs analogues hétérocycliques et leur utilisation thérapeutique**

(30) Priorité: 01.10.1993 FR 9311721
(71) Demandeur: LABORATOIRE INNOTHERA Société Anonyme, F-94110 Arcueil (FR)
(72) Inventeur: Desquand, Stéphanie, F-75015 paris (FR); Finet, Michel, F-94260 Fresnes (FR); Le Marquer, Florence, F-75005 Paris (FR); Tembo, Norbert Olivier, F-95800 Cergy Saint, Christophe (FR); Torregrosa, Jean-Luc, F-93200 Saint Denis (FR); Yannic-Arnoult, Sylvie, F-91350 Grigny (FR)
(74) Mandataire: Dupuis-Latour, Dominique

(57) **Abrégé**

La présente invention a pour objet des dérivés d'indane cétoniques et de leurs analogues hétérocycliques qui répondent à la formule générale : 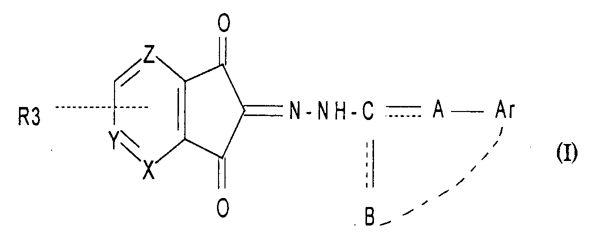
dans laquelle X, Y, Z sont indépendamment l'un de l'autre un atome de carbone ou un atome d'azote ;
B est choisi parmi un atome d'oxygène, de soufre ou un radical R₁N où R₁ est un atome d'hydrogène ou un radical alkyle en C₁ à C₅,
A est un atome d'azote ou un radical R₂N où R₂ est un atome d'hydrogène ou un radical alkyle en C₁ à C₅.
A_{R} est un cycle aromatique ou hétéroaromatique ayant un ou plusieurs hétéroatomes substitués ou non substitués,
R₃ est un atome d'hydrogène, un atome d'halogène, un radical N_{X}R₄R_{4'} où x est un entier de 1 à 2, R₄ et R_{4'} sont des radicaux organiques monovalents en C₁ à C₅ hydrogénés ou oxygénés.

## Description

La présente invention concerne les dérivés d'indane cétoniques et de leurs analogues hétérocycliques et leur utilisation thérapeutique.

Les dérivés d'indane cétoniques et de leurs analogues hétérocycliques selon l'invention répondent à la formule générale: 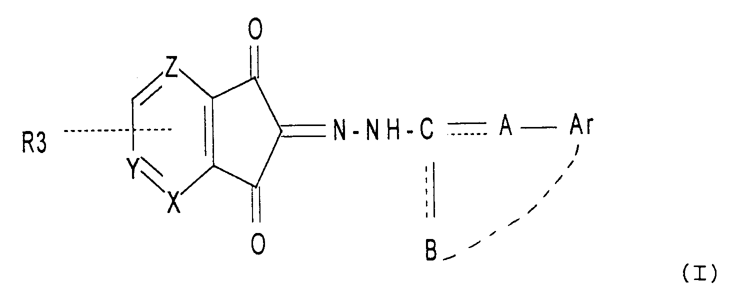
dans laquelle X, Y, Z sont indépendamment l'un de l'autre un atome de carbone ou un atome d'azote,
B est choisi parmi un atome d'oxygène, de soufre ou un radical R₁N où R₁ est un atome d'hydrogène ou un radical alkyle en C₁ à C₅,
A est un atome d'azote ou un radical R₂N où R₂ est un atome d'hydrogène ou un radical alkyle en C₁ à C₅.
Aᵣ est un cycle aromatique ou hétéroaromatique ayant un ou plusieurs hétéroatomes substitués ou non substitués,
R₃ est un atome d'hydrogène, un atome d'halogène, un radical N_{X}R₄R_{4'} où x est un entier de 1 à 2, R₄ et R_{4'} sont des radicaux organiques monovalents en C₁ à C₅ hydrogénés ou oxygénés.

Selon un mode de réalisation de la présente invention dans la formule (I) le radical Aᵣ est relié au radical B par une liaison covalente.

Selon un mode de réalisation de la présente invention dans la formule (I) X, Y et Z sont analogues et représentent un atome de carbone,
B est choisi parmi un atome d'oxygène, de soufre, un radical R₁N où R₁ représente un atome d'hydrogène,
A est un atome d'azote,
Aᵣ est un radical phényle et Aᵣ est relié à B par une liaison covalente,
R₃ est un atome d'hydrogène.

Selon un autre mode de réalisation de la présente invention dans la formule (I) X, Y et Z sont analogues et représentent chacun un atome de carbone,
B est un radical R₁N où R₁ est un atome d'hydrogène,
A est un atome d'azote,
Aᵣ est la pyridine et Aᵣ est relié à B par une liaison covalente et R₃ est un atome d'hydrogène.

Selon un autre mode de réalisation de la présente invention dans la formule (I) l'un des substituants X, Y et Z est un atome d'azote tandis que les deux autres sont des atomes de carbone,
B est un atome d'oxygène,
A est un radical R₂N où R₂ représente un atome d'hydrogène,
Aᵣ est un radical phényle et R₃ est un atome d'hydrogène.

Selon un autre mode de réalisation de la présente invention dans la formule (I) X et Z sont analogues et représentent un atome d'azote, Y est un atome de carbone, B est un atome d'oxygène, A est un radical R₂N où R₂ représente un atome d'hydrogène, Aᵣ est un radical phényle et R₃ est un atome d'hydrogène.

Selon un autre mode de réalisation de la présente invention dans la formule (I) X, Y et Z sont analogues et représentent un atome de carbone, B est un radical R₁N où R₁ est un atome d'hydrogène, A est un atome d'azote, Aᵣ est un radical phényle et Aᵣ est relié à B par une liaison covalente, R₃ est choisi parmi un atome d'halogène, un radical N_{X}R₄R₄' où x vaut 1 et R₄ et R_{4'} sont analogues et représentent chacun un atome d'oxygène.

La présente invention concerne également les sels des composés salifiables mentionnés plus haut. Ces sels comprennent les sels d'addition d'acides minéraux tels que l'acide chlorhydrique, l'acide bromhydrique, l'acide sulfurique, l'acide phosphorique ou l'acide nitrique et les sels d'addition d'acides organiques tels que l'acide acétique, l'acide propionique, l'acide oxalique, l'acide citrique, l'acide maléique, l'acide fumarique, l'acide succinique et l'acide tartrique.

Dans la présente invention, il est possible de produire soit un monosel, soit un bisel. Le bisel peut être de même nature que le monosel ou de nature différente de celui-ci.

La présente invention vise également l'utilisation des dérivés précipités pour l'obtention d'un médicament destiné au traitement de l'insuffisance veineuse fonctionnelle et organique, des pathologies hémorroïdaires, des inflammations ostéoarticulaires, dermatologiques et cardiovasculaires et des états de chocs constitués par une chute importante de la pression artérielle plus particulièrement dans les états de chocs septiques.

L'invention est illustrée par les exemples non limitatifs suivants :

### exemple 1

### 2-(Benzothiazol-2-yl-hydrazono)-2.3-dihydro-1.3-dioxo-1H-indene

A 10.8g (60.6 mmoles) de 2.3-dihydro-2.2-dihydroxy 1.3-dioxo-1H-indene en solution dans 25 ml d'éthanol, on ajoute à 5°C 10g de 2-Hydrazinobenzothiazole. Le précipité qui se forme après 10 mn de réaction est filtré sur verre fritté rincé au pentane et purifié sur Flash chromatographie avec du dichlorométhane pour fournir 8g de 2-(benzothiazol-2-yl-hydrazono)-2.3-dihydro-1.3-dioxo-1H-indene sous forme de cristaux rouges.
- **Rdt :** 43%
- **F :** 286°C
- **Rf :** 0.56 (CH₂Cl₂-MeOH, 99: 1)
- **RMN du ¹H (270 MHz, DMSO d₆) : d (ppm)**

7.89 (d, 1H, H-7', JH6'-J7'=8.5Hz)
7.80 (ls, 4H, H-4, H-5, H-6, H-7)
7.67 (d, 1H, H-4', JH4'-5' =8.5Hz)
7.40 (t, 1H, H-7', JH6'-7'=8.5Hz, JH5'-6'=7.6Hz)
7.28 (t, 1H, H-5', JH5'-6'=7.6Hz, JH4'-5'=8.5Hz)
3.55 (ls, 1H, NH)
**-RMN du ¹³C (270 MHz, DMSO d₆) : d (ppm)**
186.5 (C=O), 185.35 (C=O), 155.17 (C-2'), 152.60 (C-7'a), 139.7 (C-2, C-3a, C-7a), 136.18 (C-5, C-6), 133.20 (C-3'a), 125.1 (C-5', C-6'), 123.2 (C-4, C-7), 122.70 (C-4', C-7')
**-IR (KBr)**
ν NH : 3150 cm⁻¹, C=O : 1687cm⁻¹
**-SM (I.E.)** : m/z 307 (M^{+.})

### exemple 2

### 2-(Benzoxazol-2-yl-hydrazono)-2.3-dihydro-1.3-dioxo-1H-indene

A 5.15g (29 mmoles) de 2.3-Dihydro-2.2-dihydroxy-1.3-dioxo-1H-indene en solution dans 150 ml d'éthanol on ajoute à 5°C 4.3g de 2-Hydrazinobenzoxazol. Le précipité qui se forme après 10 mn de réaction est filtré sur verre fritté rincé au pentane et purifié sur Flash chromatographie avec un mélange CH2Cl2/Heptane, 90/10, pour fournir 4g de 2-(Benzoxazol-2-yl-hydrazono)-2.3-dihydro-1.3-dioxo-1H-indene sous forme de cristaux orange.
- **Rdt :** 47%
- **F :** >300°C
- **Rf :** 0.68 (CH₂Cl₂-MeOH, 99 : 1)
- **RMN du ¹H (270 MHz, DMSO d₆) : d (ppm)**

7.99 (s, 4H, H-4, H-5, H-6, H-7)
7.72 (d, 1H, H-4', JH4'-5' =7.48Hz)
7.59 (d, 1H, H-7', JH6'-7'=7.02Hz)
7.35 (m, 2H, H-5', H-6')
3.55 (ls, 1H, NH)
**-RMN du ¹³C (270 MHz, DMSO d₆) : d (ppm)**
186.5 (C=O), 185.35 (C=O), 148.17 (C-2'), 139.7 (C-3'a, C-7'a, C-2, C-3a, C-7a), 136.18 (C-5, C-6), 124.0 (C-5', C-6', C-7'), 123.2 (C-4, C-7), 110.54 (C-4')
**-IR (KBr)**
ν NH : 3150 cm⁻¹, C=O : 1686 et 1633cm⁻¹
**-SM (I.E.)** : m/z 291 (M^{+.})

### exemple 3

### 2-(Benzimidazol-2-yl-hydrazono)-2.3-dihydro-1.3-dioxo-1H-indene

A 6g (33.78 mmoles) de 2.3-dihydro-2.2-dihydroxy-1.3-dioxo-1H-indene en solution dans 50 ml d'éthanol on ajoute à 5°C 5g de 2-hydrazinobenzimidazole. Le précipité qui se forme après 15 minutes de réaction est filtré sur verre fritté rincé au pentane et purifié sur Flash chromatographie avec un mélange CH₂Cl₂/Heptane/MeOH, 89.55/9.95/0.5, pour fournir 4g de 2-(benzimidazol-2-yl-hydrazono)-2.3-dihydro-1.3-dioxo-1H-indene sous forme de cristaux orange.
- **Rdt :** 40%
- **F :** >260°C
- **Rf :** 0.64 (CH₂Cl₂-MeOH, 95 : 5)
- **RMN du ¹H (270 MHz, DMSO d₆) : d (ppm)**

11.25 (ls, 1H, NH)
7.80 (ls, 4H, H-4, H-5, H-6, H-7)
7.43 (m, 2H, H-4', H-7')
7.29 (m, 2H, H-5', H-6')
3.55 (ls, 1H, NH)
**-RMN du ¹³C (270 MHz, DMSO d₆) : d (ppm)**
186.5 (C=O), 185.35 (C=O), 158.32 (C-2'), 139.28 (C-2, C-3a, C-7a), 134.2 (C-5, C-6), 131.28 (C-3'a, C-7'a), 123.49 (C-4', C-7'), 121.9 (C-4, C-7), 112.18 (C-5', C-6')
**-IR (KBr)**
ν NH : 3505 et 3410cm⁻¹, C=O : 1644cm⁻¹
**-SM (I.E.)** : m/z 290 (M^{+.})

### exemple 4

### 2-(4-Azabenzimidazol-2-yl-hydrazono)-2.3-dihydro-1.3-dioxo-1H-indene

A 5g (28.08 mmoles) de 2.3-dihydro-2.2-dihydroxy-1.3-dioxo-1H-indene en solution dans 50 ml d'éthanol, on ajoute à 5°C 4.18g (28.08 mmoles) de 2-hydrazino-4-azabenzimidazole. Le précipité qui se forme après 15 mn de réaction est filtré sur verre fritté rincé au pentane et purifié sur Flash chromatographie avec un mélange CH₂Cl₂/MeOH, 95/5, pour fournir 5.3g de 2-(4-azabenzimidazol-2-yl-hydrazono)-2.3-dihydro-1.3-dioxo-1H-indene sous forme de cristaux orange.
- **Rdt :** 65%
- **F :** >260°C
- **Rf :** 0.60 (CH₂Cl₂-MeOH, 95 : 5)
- **RMN du ¹H (270 MHz, DMSO d₆) : d (ppm)**

11.65 (ls, 1H, NH)
8.70 (d, 1H, H-5', JH5'-6'=8.2Hz)
7.85 (ls, 4H, H-4, H-5, H-6, H-7)
7.38 (m, 1H, H-6')
7.13 (d, 1H, H-7', JH7'-8'=7.9Hz)
3.45 (ls, 1H, NH)
**-RMN du ¹³C (270 MHz, DMSO d₆) : d (ppm)**
186.95 (C=O), 185.19 (C=O), 157.55 (C-2'), 153.65 (C-7'a), 143.79 (C-5'), 139.25 (C-2, C-3a, C-7a), 133.49 (C-5, C-6), 131.40 (C-3'a), 120.72 (C-4, C-7), 120.45 (C-6'), 118.27 (C-7')
**-IR (KBr)**
ν NH : 3255 cm⁻¹, C=O : 1644cm⁻¹
**-SM (I.E.)** : m/z 291 (M^{+.})

### exemple 5

### 2-(5-Azabenzimidazol-2-yl-hydrazono)-2.3-dihydro-1.3-dioxo-1H-indene

A 5g (28.08 mmoles) de 2.3-dihydro-2.2-dihydroxy-1.3-dioxo-1H-indene en solution dans 50 ml d'éthanol on ajoute à 5°C 4.18g (28.08 mmoles) de 2-hydrazino-5-azabenzimidazole. Le précipité qui se forme après 15 mn de réaction est filtré sur verre fritté rincé au pentane et purifié sur Flash chromatographie avec un mélange CH₂Cl₂/MeOH, 95/5, pour fournir 5.3g de 2-(5-azabenzimidazol-2-yl-hydrazono)-2.3-dihydro-1.3-dioxo-1H-indene sous forme de cristaux orange.
- **Rdt** : 65%
- **F :** >260°C
- **Rf :** 0.54 (CH₂Cl₂-MeOH, 93: 7)
- **RMN du ¹H (270 MHz, DMSO d₆) : d (ppm)**

11.40 (ls, 1H, NH)
8.59 (ls, 1H, H-4')
8.35 (d, 1H, H-6', JH6'-7'=8.2Hz)
7.90 (s, 4H, H-4, H-5, H-6, H-7)
7.38 (d, 1H, H-7', JH6'-7'=8.2Hz)
3.60 (ls, 1H, NH)
**-RMN du ¹³C (270 MHz, DMSO d₆) : d (ppm)**
186.35 (C=O), 185.65 (C=O), 159.43 (C-2'), 154.95 (C-7'a), 144.85 (C-4'), 139.64 (C-2, C-3a, C-7a), 133.55 (C-5, C-6), 128.40 (C-3'a), 125.3 (C-6'), 120.72 (C-4, C-7), 120.34 (C-7')
**-IR (KBr)**
ν NH : 3250 et 3410cm⁻¹, C=O : 1665cm⁻¹
**-SM (I.E.)** : m/z 291 (M^{+.})

### exemple 6

### 5.7-Dioxo-6-(phenylaminocarbonylhydrazono)cyclopenta[b]pyridine

A une solution de 1g (5.6mmoles) de 6.6-dihydroxy-5.7-dioxo-cyclopenta[b]pyridine dans 5ml d'éthanol, on ajoute à 50°C une solution fraîchement préparée contenant 0.84g (5.6mmoles) de phénylaminocarbonylhydrazine et 5ml d'eau.

Le mélange réactionnel est agité à température ambiante pendant une nuit et le précipité marron formé est essoré puis purifié sur colonne.(éluant : CH2Cl2/Hep. : 98/2)
Cristaux orange
**Rdt :** 30%
**F :** déc. 145°C
**SM :** (I.E) : m/z 282 (M^{+.})
**RMN du ¹H (270 MHz, DMSO d₆) : d (ppm)**
13.14 (ls, 1H, NH)
12.83 (ls, 1H, NH)
9.26 (d, 1H, H-2, J H2-3 = 6Hz)
9.00 (d, 1H, H-4, J H2-3 = 6Hz)
8.80 (m, 1H, H-3)
7.65 (m, 2H, H-2', H-6' )
7.41 (m, 2H, H-3', H-5' )
7.20 (m, 1H, H-4')
**-SM (I.E.)** : m/z 294 (M^{+.})
**IR (KBr)**
ν NH : 3220 cm⁻¹, C=O : 1748 et 1690 cm^{-1.}

### exemple 7

### 5.7-Dioxo-6-(phenylaminocarbonylhydrazono)cyclopenta[c]pyridine

A une solution de 1g (5.6mmoles) de 6.6-dihydroxy-3.5-dioxo-cyclopenta[c]pyridine dans 5ml d'éthanol, on ajoute à 50°C une solution fraichement préparée contenant 0.84g (5.6mmoles) de phénylaminocarbonylhydrazine, 5ml d'eau et 3 gouttes d'acide chlorhydrique concentré.

Le mélange réactionnel est agité à température ambiante pendant une nuit et le précipité marron formé est essoré puis purifié sur colonne d'aluminum.
(éluant : CH2Cl2/Hep. : 98/2)
Cristaux orange
**Rdt :** 40%
**F :** déc 140°C
**SM :** (I.E) : m/z 282 (M^{+.})
**RMN du ¹H (270 MHz, DMSO d₆) d (ppm)**
12.85 (ls, 1H, NH)
11.42 (ls, 1H, NH)
9.26 (s, 1H, H-2)
8.56 (d, 1H, H-7, JH6-7=6Hz)
7.80 (d, 1H, H-6, JH6-7=6Hz)
7.71 (m, 2H, H-2', H-6' )
7.36 (m, 2H, H-3', H-5' )
7.18 (m, 1H, H-4')
**-SM (I.E.)** : m/z 294 (M^{+.})
**IR (KBr)**
ν NH : 3220 cm⁻¹, C=O : 1748 et 1690 cm^{-1.}

### exemple 8

### 5.7-Dioxo-6-(phénylaminocarbonylhydrazono)cyclopentapyrazine

A une solution de 1g(5.6mmoles) de 6.6-dihydroxy-5.7-dioxo-cyclopentapyrazine dans 5ml d'éthanol,on ajoute à 50°C une solution fraichement préparée contenant 0.84g (5.6mmoles) de phénylaminocarbonylhydrazine, 5ml d'eau et 3 gouttes d'acide chlorhydrique concentré.

Le mélange réactionnel est agité à température ambiante pendant une nuit et le précipité marron formé est essoré puis purifié sur colonne d'alumine.
(éluant: CH2Cl2/Hep. : 98/2)
Cristaux orange
**Rdt :** 30%
**F :** déc. 145°C
**Rf :** 0.54 (CH₂Cl₂-MeOH, 93: 7)
**RMN du ¹H (270 MHz, DMSO d₆) : d (ppm)**
13.14 (ls, 1H, NH)
12.83 (ls, 1H, NH)
8.55 (d, 2H, H-2, H-3, J H2-3 = 6Hz)
7.65 (m, 2H, H-2', H-6' )
7.41 (m, 2H, H-3', H-5' )
7.20 (m, 1H, H-4')
**SM** (I.E) : m/z 295 (M^{+.})
**IR (KBr)**
ν NH : 3220 cm⁻¹, C=O : 1748 et 1690 cm^{-1.}

### exemple 9

### 2-(Benzimidazol-2-yl-hydrazono)-2,3-dihydro-1,3,dioxo-4-fluoro-1H-indene

A 5g (25,51 mmoles) de 2,3-dihydro-2,2-dihydroxy-1,3-dioxo-4-fluoro-1H-indene en solution dans 50 ml d'éthanol, on ajoute à 5°C, 3,77 g (25,51 mmoles) de 2-hydrazinobenzimidazole.
Le précipité qui se forme après 15 mn de réaction est filtré sur verre fritté rincé au pentane et purifié sur Flash chromatographie avec un mélange CH₂Cl₂/MeOH, 94/6, pour fournir 4,3 g de 2-Benzimidazol-2-yl-hydrazono)2-3-dihydro-1,3-dioxo-4-fluoro-1H-indene sous forme de cristaux orangés.
- **Rdt :** 55%
- **Rf:** >260°C
- **Rf:** 0,55 (CH₂Cl₂-MeOH, 96:4)
- **RMN du ¹H (270 MHz, DMSO d₆) δ (ppm)**

11,78 (1s, 1H, NH)
8,04 (m, 1H, H-7)
7,80 (m, 2H, H-4, H-6)
7,53 (m, 2H, H-4', H-7')
7,31 (m, 2H, H-5', H-6')
4,65 (1s, 1H, NH)
**-IR (KRr)**
ν NH: 3505 et 3410 cm⁻¹, C=O : 1656 cm⁻¹
**-SM (I.E.) :** m/z 3,08 (M⁺.)

### exemple 10

### 2-(Benzimidazol-2-yl-hydrazono)-2,3-dihydro-1,3-dioxo-4-nitro-1H-indene

A une solution de 2,23 g (0,01 mole) de 2,3-dihydro-2,2-dihydroxy-1,3-dioxo-4-nitro-1H-indene dans 50 ml d'éthanol, on ajoute à 5°C, 1,48 g (0,01 mole) de 2-hydrazinobenzimidazole.
Le précipité qui se forme après 15 minutes de réaction est filtré sur verre fritté, rincé au pentane et purifié sur Flash chromatographie avec un mélange CH₂Cl₂/MeOH, 99/1, pour fournir 2g de 2-(Benzimidazol-2-yl-hydrazono)-2,3-dihydro-1,3-dioxo-4-nitro-1H-indene sous forme de cristaux orangés.
- **Rdt:** 61%
- **F:** > 260°C
- **Rf: 055 (CH₂Cl₂-MeOH, 96:4)**
- **RMN du ¹H (270 MHz, DMSO d₆: δ (ppm)**

11,78 (ls, 1H, NH)
8,33 (d, 1H, H-5, J H5-6 = 7,32Hz)
8,22 (d, 1H, H-7, J H7-6 = 7,32Hz)
8,05 (t, 1H, H-6, J H6-7 = 7,32Hz)
7,53 (m, 2H, H-4', H-7')
7,31 (m, 2H, H-5', H-6')
4,65 (ls, 1H, NH)
**IR (KBr)**
ν NH : 3505 et 3410 cm⁻¹, C=O : 1656 cm⁻¹
**-SM (I.E.)** : m/z 335 (M⁺.)

L'étude des composés de la présente invention et de leurs sels a montré qu'ils possèdent diverses propriétés pharmacologiques. Ainsi, ils sont veinotoniques et n'affectent pas dans la plupart des cas le système artériel ; par ailleurs, ils augmentent la résistance capillaire, diminuent l'hyperperméabilité vasculaire induite par certains agents inflammatoires et présentent des propriétés antilipoperoxydantes, antiradicalaires et antiinflammatoires, ainsi qu'une activité protectrice dans le choc septique.

Ces propriétés sont démontrées chez les mammifères tels que les rats, cobayes et lapins, dans des conditions in vitro (vaisseaux ou réseaux vasculaires isolés) et in vivo.

Pour l'étude in vitro, les composés sont solubilisés en solution aqueuse pure ou contenant du DMSO ou de l'alcool.
Pour l'étude in vivo, ils sont administrés par voie intra-veineuse sous forme de solution aqueuse pure, par voie intrapéritonéale sous forme de solution aqueuse contenant ou non du DMSO ou par voie orale en solution ou en suspension dans la carboxyméthylcellulose ou dans une solution aqueuse composée contenant du Tween(R) et dans certains cas du DMSO (diméthylsulfoxyde).

### Modèles d'études pharmacologiques

L'effet contractile est mesuré in vitro :
- par la force de contraction développée par des anneaux vasculaires quiescents ou stimulés (soit électriquement soit par des agents physiologiques) et maintenus dans des conditions isométriques,
- par la pression développée par des réseaux vasculaires perfusés à débit constant.

In vivo, les pressions artérielles et veineuses sont mesurées dans des conditions normales et après arrêt cardiaque. Lors de l'arrêt cardiaque, le tonus veineux est calculé à partir des pressions veineuses et artérielles mesurées à l'équilibre et corrigées en fonction des différences relatives de compliance entre ces deux réseaux (Samar et Coleman, Am. J. Physiol., 1978, 234 : H94-100 ; Yamamoto et col., Am. J; Physiol., 1980, 238 : H823-828).

L'augmentation de la résistance capillaire est appréciée par la modification de l'index pétéchial (pression négative induisant l'extravasation d'érythrocytes), mesuré par une méthode dérivée de l'angiosterromètre de Parrot.

La perméabilité vasculaire est étudiée in vivo et in vitro par la mesure de l'extravasation d'albumine ou de colorant liant l'albumine (Bleu Evans). In vivo, l'hyperperméabilité est induite par l'injection d'une solution d'histamine, de bradykinine ou de zymosan. Les modèles in vitro permettent de réaliser des hyperpressions (sur un territoire vasculaire isolé) et/ou des réactions vasculaires inflammatoires.

L'activité antiinflammatoire est démontrée par la mesure de l'inhibition de l'oedème et de la migration leucocytaire après induction d'une pleurésie chez le rat par injection de carragénine dans la cavité pleurale (Almeida et col., J. Pharmacol. Exp. Therap., 1980, 214 : 74).

L'effet "piégeur de radicaux libres" global est étudié in vitro par un modèle utilisant le 1,1-diphényl-2-picrylhydrazyl (DPPH) comme radical libre stable, méthode dérivée de celle décrite par Lamaison et col., Plantes Médic. et Phytothérapie, XXII, 1988, 231-234.

L'activité antioxydante est étudiée in vitro par un modèle de peroxydation lipidique basée sur la peroxydation d'une émulsion d'acide linoléique par le fer, méthode modifiée par rapport à celle décrite par Sutherland et col., Arch. Biochem. Biophys., 1982, 214, 1-11.

L'activité dans le choc septique est étudiée chez le rat après induction par une endotoxine lipopolysaccharidique (15 mg/kg), méthode voisine de celle décrite par Terashita et col., Eur. J. Pharmacol., 109, 257-261, 1985.

### Exemples d'effets pharmacologiques

Les composés de formule (I) et leurs sels éventuels augmentent la contraction des veines saphènes animales produite par la noradrénaline et la dépolarisation (solution hyperpotassique) sans affecter, dans la majorité des cas, les réponses contractiles artérielles. A titre illustratif, les composés des exemples 1, 2, 3, 5 (10 nM à 30 mM) augmentent de plus de 40 % (DE₅₀ + 0,3 microM) les contractions des veines saphènes de lapin produites par le KCl (40 mM).

Les composés des exemples 1, 4 et 6 augmentent à leur concentration maximale de 30 à 200 % la contraction des veines saphènes de lapin en réponse à la noradrénaline 0,3 micromolaire.

A titre illustratif, les composés des exemples 12, 3, 5 augmentent la résistance capillaire de base de 10 à 100 %, lorsque celle-ci est mesurée une heure à deux heures après administration de 1-20 mg/kg/i.p. et jusqu'à quatre à six heures après administration orale de 1-20 mg/kg chez le rat.

A titre illustratif, les composés des exemples 1, 2, 3, 5 inhibent l'hyperperméabilité vasculaire induite par le zymosan après administration orale de 1-20 mg/kg.

Le composé des exemples 31, 3, 5 administrés deux fois en i.p. à la dose de 5 mg/kg inhibent l'oedème et la migration leucocytaire dans la cavité pleurale, 6 heures après injection de carragénine dans le modèle de la pleurésie chez le rat.

A titre illustratif, les composés des exemples 2, 6 présentent un effet antiradicalaire dans le modèle utilisant le DPPH.

### Toxicité

Par ailleurs, les composés de formule (I) et leurs sels éventuels sont très peu toxiques. Par exemple, après administration unique per os chez la souris, aucune mortalité n'est observée à la dose de 1 g/kg pour la majorité des composés. Les seuls effets observés sont dans certains cas des diarrhées colorées et des urines colorées, ces dernières étant le témoin d'une résorption du produit.

Ce qui précède montre que les composés de formule (I) et leurs sels éventuels peuvent être utilisés en thérapeutique humaine et animale. Ils sont en particulier indiqués dans l'insuffisance veineuse fonctionnelle, organique et les pathologies hémorroïdaires par leurs composantes vasculaires et antiinflammatoires, ainsi que dans les affections typiquement inflammatoires (ostéoarticulaires, dermatologiques ou cardiovasculaires) et dans les états de chocs constitués par une chute importante de la pression artérielle, en particulier dans les états de chocs septiques (endotoxiques).
L'insuffisance veineuse fonctionnelle se caractérise par une dilatation et une hyperdistensibilité des veines superficielles des membres inférieurs, oedèmes, paresthésies à type d'impatince, de jambe sans repos. Ce type de pathologie peut évoluer vers l'insuffisance veineuse organique (varices, incontinence valvulaire profonde ...) voire vers la phlébothrombose et les lésions ulcéreuses.

Dans cette pathologie veineuse, une composante inflammatoire s'installe dans les premiers stades et se manifeste plus clairement dans les stades avancés.

La présente invention comprend donc l'utilisation des composés de formule (I) ci-dessus décrits et de leurs sels éventuels, comme substances actives pour la préparation de médicaments et compositions pharmaceutiques, à usage humain et vétérinaire, comprenant au moins un desdits composés et sels en association avec un support ou diluant physiologiquement acceptable.

La forme de ces médicaments et compositions pharmaceutiques dépendra bien évidemment de la voie d'administration souhaitée notamment orale, parentérale et rectale et ils peuvent être formulés selon les techniques classiques avec mise en oeuvre des supports et véhicules usuels.

Ainsi, dans le cas d'une administration par voie orale, ils peuvent se présenter sous la forme de comprimés, tablettes, gélules, solutions, sirops et suspensions.

Les comprimés, tablettes et gélules contiennent la substance active conjointement avec un diluant (par exemple lactose, dextrose, sucrose, mannitol, sorbitol ou cellulose), un lubrifiant (par exemple silice, talc ou stéarate comme le stéarate de magnésium), un liant (par exemple amidon, méthylcellulose ou gomme arabique), un agent de désintégration (alginate par exemple) et ils sont fabriqués par des techniques connues par exemple de mélange, de granulation, de pastillage, d'enrobage, etc ...

Les sirops peuvent contenir, à titre de support, glycérol, mannitol et/ou sorbitol. Les solutions et suspensions peuvent comprendre de l'eau et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support approprié tel que l'eau stérile ou des solutions salines isotoniques aqueuses stériles.

Pour l'administration par voie rectale, ils peuvent prendre la forme de suppositoires comprenant la substance active et un support approprié tel que le beurre de cacao ou du polyéthylène glycol.

La dose thérapeutique des substances actives pourra atteindre 2000 mg/jour suivant la voie d'administration, l'âge, le poids et l'état du sujet à traiter et la puissance thérapeutique de la substance active mise en oeuvre.

## Revendications

1. Dérivés d'indane cétoniques et de leurs analogues hétérocycliques, caractérisés en ce qu'ils répondent à la formule générale : 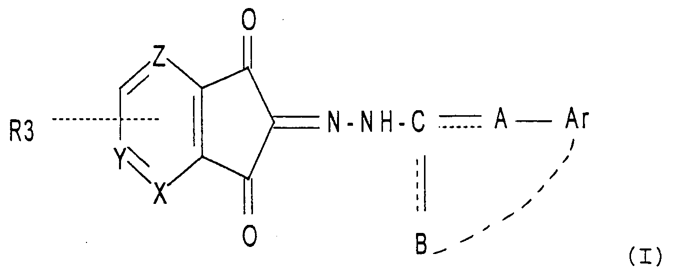 dans laquelle X, Y, Z sont indépendamment l'un de l'autre un atome de carbone ou un atome d'azote,
B est choisi parmi un atome d'oxygène, de soufre ou un radical R₁N où R₁ est un atome d'hydrogène ou un radical alkyle en C₁ à C₅,
A est un atome d'azote ou un radical R₂N où R₂ est un atome d'hydrogène ou un radical alkyle en C₁ à C₅,
Aᵣ est un cycle aromatique ou hétéroaromatique ayant un ou plusieurs hétéroatomes substitués ou non substitués,
R₃ est un atome d'hydrogène, un atome d'halogène, un radical nitro ou un radical N_{X}R₄R₄, où x est un entier de 1 à 2 et R₄ et R₄, sont des radicaux organiques monovalents en C₁ à C₅.

2. Dérivés selon la revendication 1, caractérisés en ce que le radical Aᵣ est relié au radical B par une liaison covalente.

3. Dérivés selon l'une des revendications 1 ou 2, caractérisés en ce que X, Y et Z sont analogues et représentent un atome de carbone,
B est choisi parmi un atome d'oxygène, de soufre, un radical R₁N où R₁ représente un atome d'hydrogène,
A est un atome d'azote,
Aᵣ est un radical phényle et Aᵣ est relié à B par une liaison covalente, et
R₃ est un atome d'hydrogène.

4. Dérivés selon la revendication 1, caractérisés en ce que X, Y et Z sont analogues et représentent chacun un atome de carbone,
B est un radical R₁N où R₁ est un atome d'hydrogène,
A est un atome d'azote,
Aᵣ est la pyridine et Aᵣ est relié à B par une liaison covalente et R₃ est un atome d'hydrogène.

5. Dérivés selon la revendication 1, caractérisés en ce que l'un des substituants X, Y et Z est un atome d'azote tandis que les deux autres sont des atomes de carbone,
B est un atome d'oxygène
A est un radical R₂N où R₂ représente un atome d'hydrogène,
Aᵣ est un radical phényle et R₃ est un atome d'hydrogène.

6. Dérivés selon la revendication 1, caractérisés en ce que X et Z sont analogues et représentent un atome d'azote, Y est un atome de carbone, B est un atome d'oxygène, A est un radical R₂N où R₂ représente un atome d'hydrogène, Aᵣ est un radical phényle et R₃ est un atome d'hydrogène.

7. Dérivés selon la revendication 1, caractérisés en ce que X, Y et Z sont analogues et représentent un atome de carbone, B est un radical R₁N où R₁ est un atome d'hydrogène, A est un atome d'azote, Aᵣ est un radical phényle et relié à B par une liaison covalente et R₃ est choisi parmi un atome d'halogène ou un radical nitro.

8. Les sels d'addition d'acides minéraux choisis parmi l'acide chlorhydrique, l'acide bromhydrique, l'acide sulfurique, l'acide phosphorique et l'acide nitrique et les sels d'addition d'acides organiques choisis parmi l'acide acétique, l'acide propionique, l'acide oxalique, l'acide citrique, l'acide maléique, l'acide fumarique, l'acide succinique et l'acide tartrique des composés selon l'une quelconque des revendications 1 à 7.

9. Utilisation des composés selon l'une quelconque des revendications 1 à 8 pour l'obtention d'un médicament destiné au traitement de l'insuffisance veineuse fonctionnelle et organique.

10. Utilisation des composés selon l'une quelconque des revendications 1 à 8 pour l'obtention d'un médicament destiné au traitement des pathologies hémorroïdaires.

11. Utilisation des composés selon l'une quelconque des revendications 1 à 8 pour l'obtention d'un médicament destiné au traitement des inflammations ostéoarticulaires dermatologiques et cardiovasculaires.

12. Utilisation des composés selon l'une quelconque des revendications 1 à 8 pour l'obtention d'un médicament destiné au traitement des états de chocs constitués par une chute importante de la pression artérielle plus particulièrement dans les états de chocs septiques.
